# EUROPEAN PATENT APPLICATION

(11) **EP 2 399 613 A1**
(43) Date of publication of application: **28.12.2011**
(21) Application number: 10305669.3
(22) Date of filing: 22.06.2010
(51) Int. Cl.: A61L 2/00, C07K 16/06

(54) **Fractionation of plasma using caprylic acid**

(71) Applicant: Research Foundation For Medical Devices, 1700 Fribourg (CH)
(72) Inventor: Burnouf, Thierry, 59249, AUBERS (FR); El-Ekiaby, Magdy, CAIRO (EG); Goubran, Hadi Alphonse, 11431, CAIRO (EG); Radosevich, Miryana, 59249, AUBERS (FR)
(74) Representative: Cabinet Plasseraud

(57) **Abstract**

The present invention relates to a process of immunoglobulin G fractionation using caprylic acid.

## Description

The present invention concerns the small-pool fractionation of Immunoglobulin g using caprylic acid.

### Background of the Invention

Human and animal blood plasma and plasma fractions play an important role in modem medicine. They are mainly used for transfusion purposes in the treatment of bleeding episodes, thrombotic episodes, fibrinolysis, infectious episodes, or for prophylaxis or for pre-treating patients prior to surgery in certain clinical situations.

A major drawback in the use of human or animal blood plasma or plasma fractions is the risk of transmission of blood borne viruses, such as, in the case of plasma from human origin, human immunodeficiency virus (HIV), hepatitis B virus (HBV), hepatitis C virus (HCV), and West Nile Virus, or possibly emerging viruses such as Severe Acute Respiratory Syndrome (SARS) virus, and avian flu virus, which are all lipid-enveloped viruses. Viral risks also exist from products obtained from recombinant mammalian cell lines and from transgenic animals.

Therefore, different methods for viral inactivation of blood plasma and/or plasma fractions and/or recombinant products have been developed, such as the treatment with methylene blue followed by irradiation with visible light and solvent/detergent or solvent alone treatment. US patent Nos. 4,481,189 and 4,540,573 describe for example the use of organic solvent detergent combinations (S/D method) or solvent alone to reduce by several orders of magnitude the infectivity of hepatitis viruses or other enveloped viruses contained in plasma and plasma products or added thereto. Other methods of viral inactivation include pasteurisation defined as a heat treatment of a solution usually at 60°C for 10 hrs, or dry-heat treatment at various temperatures for variable duration of a freeze-dried product (Burnouf and Radosevich, Blood Rev 2000, 14: 94-110)..

Solvent/detergent or solvent alone treatment under appropriate conditions of reagent concentration, temperature and contact time effectively disassembles viruses that have envelope proteins associated with lipids, while having negligible effect on the molecular conformations and biological activity of sensitive plasma proteins.

The treatment preserves the functional activities of a wide range of coagulation factors and other labile plasma proteins with an excellent record of virucidal efficiency for the pathogenic lipid-enveloped blood-borne viruses (Burnouf and Radosevich , Blood Rev 2000, 14: 94-110).

Recently, the present inventors have developed a solvent/detergent process applied to minipools of plasma (MP/SD). In this process, which is described in international patent application WO 2006/082115, small volumes of plasma are treated in a closed bag system.

Polyvalent intravenous immunoglobulins G (IVIG) and intramuscular IgG (IM) are plasma-derived products of primary importance for the treatment of immune-deficient patients (Toubi E, Etzioni A: Intravenous immunoglobulin in immunodeficiency states: state of the art. Clin Rev Allergy Immunol. 2005;29: 167-72). In the last few years, IVIG has also increasingly become a first-line therapy against several immune-mediated inflammatory, rheumatic, and neurological disorders (Kivity S, Katz U, Daniel N, Nussinovitch U, Papageorgiou N, Shoenfeld Y: Evidence for the use of intravenous immunoglobulins--a review of the literature. Clin Rev Allergy Immunol. 2010;38: 201-69). Hyperimmune IgG, produced from plasmas selected for their high content in clinically important antibodies are used to treat or prevent specific viral and bacterial infections such as hepatitis B or tetanus, as well as haemolytic anemia of the rhesus-negative newborn. IgG, most particularly polyvalent IVIG, has become in the last few years the leading product manufactured by the fractionation of human plasma (Burnouf T: Modem plasma fractionation. Transfus Med Rev. 2007;21: 101-17). The World Health Organization has recently included IgG on the Model List of Essential Medicines, recognizing their role in addressing priority health care requirements of given populations and stressing the importance of ensuring their availability within the health system of all nations. However, although the plasma fractionation industry has made dramatic efforts to increase the recovery of IgG (Burnouf T: Modem plasma fractionation. Transfus Med Rev. 2007;21: 101-17) and increase product output from a total of about 25 million L of plasma fractionated worldwide (Burnouf T: [Plasma fractionation in the world: Current status.]. Transfus Clin Biol. 2007;14: 41-50) the IgG supply does not meet global demand, leaving many immune-deficient patients, in particular in developing countries, untreated. The development of IgG indications in auto-immune and inflammatory diseases, as well as possibly against Alzheimer disease (Dodel R, Neff F, Noelker C, Pul R, Du YS, Bacher M, Oertel W: Intravenous Immunoglobulins as a Treatment for Alzheimer's Disease Rationale and Current Evidence. Drugs. 2010;70: 513-28) may further increase supply issues.

It seems unlikely that the plasma fractionation industry will be in a position to further increase significantly the IgG recovery using the current core plasma fractionation technologies. Considering the shrinking number of whole blood donors in many developed countries, improving the availability of IgG preparations worldwide would therefore require either to increase the collection of apheresis plasma or to fractionate, through contract fractionation or domestic facilities, the excess recovered plasma currently available in some developing countries (Burnouf T: [Plasma fractionation in the world: Current status.]. Transfus Clin Biol. 2007;14: 41-50). The first approach is technically feasible but, considering the cost of apheresis plasma, can hardly be cost-effective if IVIG is the only product extracted from such expensive incremental plasma volume. The second approach, when justified, requires several years for implementation in most countries to generate a sufficient steady volume of quality plasma meeting the scale requirements of contract or domestic fractionators.

Moreover, the worldwide shortage of immunoglobulin G (IgG) is affecting, among others, patients in developing countries where such preparations are either not available in sufficient amounts or not affordable. In addition, it is a matter of fact that substitutive therapy in immunodeficient patients is best achieved using IgG preparations made from local plasma collected from donors exposed to pathogens from the same environment. Therefore, the development of an efficient and robust technology to prepare IgG from a small volume of plasma - without the need to build, qualify, validate and operate a sophisticated manufacturing pharmaceutical facility - should be of interest to increase the access to these essential medicinal products to some countries and patients in the world.

Caprylic (octanoic) acid precipitation has been shown at laboratory scale in the past to be a useful technique to precipitate non-Ig molecules from human and animal plasma (Habeeb AF, Francis RD: Preparation of human immunoglobulin by caprylic acid precipitation. Prep Biochem. 1984;14: 1-17 ; Steinbuch M, Audran R: [Isolation of IgG immunoglobulin from human plasma using caprylic acid]. Rev Fr Etud Clin Biol. 1969;14: 1054-8). Recently, caprylic acid precipitation, using different caprylic acid content and pH, has been introduced by several plasma fractionators in the industrial human plasma fractionation process as a polishing step of pre-purified IgG or IgM-enriched preparations made from precipitate II+III (Parkkinen J, Rahola A, von Bonsdorff L, Tolo H, Torma E: A modified caprylic acid method for manufacturing immunoglobulin G from human plasma with high yield and efficient virus clearance (Vox Sang. 2006;90: 97-104 ; Lebing W, Remington KM, Schreiner C, Paul HI: Properties of a new intravenous immunoglobulin (IGIV-C, 10%) produced by virus inactivation with caprylate and column chromatography. Vox Sang. 2003;84: 193-201), precipitate II (Dichtelmuller H, Rudnick D, Kloft M: Inactivation of lipid enveloped viruses by octanoic acid treatment of immunoglobulin solution. Biologicals. 2002;30: 135-42), or precipitate III (Dichtelmuller above). Interestingly, 5%/pH5.5 caprylic acid precipitation of whole plasma is a step currently used in the medium-scale (100-200L) fractionation of licensed intact hyperimmune immunoglobulin preparations including antivenoms produced from the plasma of immunized horses (Rojas G, Jimenez JM, Gutierrez JM: Caprylic acid fractionation of hyperimmune horse plasma: description of a simple procedure for antivenom production. Toxicon. 1994;32: 351-63). Such antivenom immunoglobulin preparations have been shown to exhibit high purity and to have a low protein aggregate content presumably because the immunoglobulins are never precipitated during such a fractionation process. The efficacy and safety profiles of such intravenous caprylic acid-fractionated antivenom immunoglobulins have been demonstrated in clinical trials in envenomed patients (Otero-Patino Ret al: A randomized, blinded, comparative trial of one pepsin-digested and two whole IgG antivenoms for Bothrops snake bites in Uraba, Colombia. The Regional Group on Antivenom Therapy Research (REGATHER). Am J Trop Med Hyg. 1998;58: 183-9; Otero R, et al.: A randomized blinded clinical trial of two antivenoms, prepared by caprylic acid or ammonium sulphate fractionation of IgG, in Bothrops and Porthidium snake bites in Colombia: correlation between safety and biochemical characteristics of antivenoms. Toxicon. 1999;37: 895-908).

### Description of the Invention

After long and intensive research, the inventors have now found surprisingly and unexpectedly that it was possible to produce immunoglobulin G (IgG) in a non expensive and very easy to implement way.

One subject of the invention is thus a process for obtaining immunoglobulin G (IgG) comprising the steps of:
a/ treating cryo-poor plasma, preferably human cryo-poor plasma, with caprylic acid ;
b/ centrifugation of the mixture of step a/ and recovery of the supernatant
c/ removing caprylic acid by diafiltration/concentration from the supernatant of step b/;
d/ filtrating by gravity the product obtained in step c/ above.

In one particular embodiment, the process of the invention comprises, before step a/, a step of incubating cryo-poor plasma with a weak anion exchanger. In this embodiment, the caprylic acid treatment step is carried out on the supernatant recovered after the incubation with the anion exchanger.

The process according to this embodiment thus comprises the steps of
- incubating cryo-poor plasma, preferably human cryo-poor plasma, with an anion exchanger;
- treating the supernatant recovered in the previous step with caprylic acid;
- centrifugation of the caprylic acid mixture and recovery of the supernatant
- removing caprylic acid by diafiltration/concentration from the supernatant of the previous step;
- filtrating by gravity the product obtained in the previous step.

During the incubation of the cryo-poor plasma, the coagulation factors of the PCC are adsorbed on the anion exchanger and can processed separately after separation of the anion exchanger from the supernatant.

Suitable anion exchangers for this embodiment include, but are not limited to, DEAE-Sephadex A-50, QAE-Sephadex, and DEAE-cellulose. For the purpose of the invention, DEAE-Sephadex A-50 is preferred since it is relatively cheap and disposable.

In one embodiment, the ratio of anion exchanger/cryo-poor plasma in the first step is 1 to 4 g anion exchanger/L of cryo-poor plasma, preferably 3 g/L. The incubation is preferably carried out for 15 to 60 min at a temperature of 15 to 35 °C, more preferably for about 30 min at about 31 °C.

Thanks to the process according to the invention, it is possible to obtain IgG of a good purity with a high yield. The mean recovery of IgG is of 50 to 60% by weight with respect to the IgG content of the cryo-poor plasma, corresponding to about 5 to 6 g/L of whole plasma.

The caprylic acid treatment step of the process of the invention has a double function: (1) precipitation of non-immunoglobulin molecules and viral inactivation of the IgG fraction. This step is advantageously carried out at a pH between 4.7 and 6, preferably at a pH between 5 and 5.8, more preferably at pH 5.5. Below pH 4.7, and more particularly below pH 4.5 immunoglobulins start precipitating and the supernatant develops turbidity and opalescence which impairs the subsequent diafiltration/concentration step and impairs the capacity to perform filtration by gravity. Above pH 6, the precipitation of plasma proteins is incomplete, hence leading to a decrease in purity of the IgG supernatant. Moreover, working in a pH range below pH 6 ensures efficient viral inactivation of viruses potentially present in the the IgG preparation, in particular with respect to HIV, BVDV and PRV, and all other enveloped viruses

The concentration of caprylic acid is between 4.8 and 5.2, preferably 5% (v/v). Said step is carried out at a constant temperature of between 15 and 37 °C, preferably at about 31 °C under gentle stirring.

The duration of the caprylic acid treatment step depends on the amount of product to be treated. In a preferred embodiment, the caprylic acid treatment step is carried out in a standard transfusion bag closed bag system under continuous gentle transversal agitation in a thermostated shaker incubator, preferably at pH 5.5 using 5% caprylic acid.

According to an embodiment of the invention, the diafiltration step is carried out on a sterile hemodialyser. The molecular weight cut-off of the hemodialyser is advantageously of 100kDa or less.

Preferably, the hemodialyser is a single-use or disposable hemodialyser. Suitable hemodialysers include, but are not limited to, the F4 and F6HPS models from Fresenius, Bad Homburg, Germany.

The advantage of using such a disposable dialyzer includes ease-of-use, worldwide availability as medical device, absence of cleaning validation, absence of risks of crossed contamination between batches, as well as competitive cost (about US$10 a unit) compared to the tangential flow filtration systems (several thousand US$) available for the biotechnology/plasma fractionation industry. Using such hemodialyzer, the concentration of the IgG fraction was found to be fast (typically 120 min to concentrate 4 L of IgG down to 0.8L) and uneventful, yielding a clear or slightly opalescent solution essentially free of particles or insolubles.

Thanks to the process according to the invention, the product obtained after step c/ is very homogeneous and deprived of agglomerates, and thus can further be filtrated by gravity. In case particles can be observed, the material can be subjected to a centrifugation to pellet the particles.

The concentrate obtained at the diafiltration step is then subjected to filtration by gravity. This step is preferably carried out on a pyrogen-free adsorption/clarifying capsule filter, such as the BC0025L60SP03A adsorption/clarifying capsule filter from 3M/Cuno, connected to a sterilizing filter with a pore size of 0.1 to 0.2 µm. The use of the adsorption/clarifying capsule filter was found to be essential to allow high-capacity sterile filtration of the IgG on a 0.2 µm or even 0.1 µm filter by gravity. In fact, over 1 L of IgG solution could be filtered on a 20 cm² sterile filter with a pore size of 0.2 µm. A suitable sterile filter is, for example, the 0.2 µm mini-kleenpack sterilizing filter from Pall Corporation, Dreieich, Germany. Another advantage of the adsorption/clarifying capsule filter is its capacity to further reduce any residual caprylic acid present after the dialysis step down to low or undetectable levels. The final product recovered in this step contains at least 95% by weight of IgG.

The final IgG preparation can be kept liquid at 2-8°C either at neutral pH or at pH 5.4-5.7, or at pH 4.5-5.0: under these conditions, the product remained stable and clear with no evidence of destabilization. This result is consistent with the experience gathered with caprylic acid fractionated intravenous antivenom immunoglobulins which are formulated liquid at neutral pH with a shelf life up to 3 years at 2-8°C (WHO: WHO Guidelines for the Production, Control and Regulation of Snake Antivenom Immunoglobulins. Geneva, World Health Organization, 2010). Viral safety of such mini-pool IgG preparation is of crucial importance and should rely on the implementation of the various well-established measures ensuring the safety of plasma for fractionation (WHO: *Recommendations for the production, quality control and regulation of plasma for fractionation. http:llwww.who.intlbloodproducts;* 2005; Burnouf T, Radosevich M: Reducing the risk of infection from plasma products: Specific preventative strategies. Blood Rev. 2000;14: 94-110) including proper donor screening, serological testing of donations, and when technically and economically feasible single-donor multiplex NAT testing for HIV, HBV and HCV, as performed in our center (El Ekiaby M, Lelie N, Allain JP: Nucleic acid testing (NAT) in high prevalence-low resource settings. Biologicals. 2010;38: 59-64). In addition, the manufacturing process should include one preferably two steps established to robustly inactivate the major plasma-borne pathogenic viruses (Burnouf T, Radosevich M: Reducing the risk of infection from plasma products: Specific preventative strategies. Blood Rev. 2000;14: 94-110 ; WHO: *Guidelines on viral inactivation and removal procedures intended to assure the viral safety of human blood plasma products. www.WHO.intlbloodproducts.* Geneva; 2003).

In one embodiment, the process of the invention further comprises a step e/ of virally inactivating the IgG obtained in step d/. Good results in terms of MSD distribution and physical stability were obtained with a pH4 incubation, a robust viral inactivation method mostly for lipid-enveloped viruses (Burnouf T, Radosevich M: Reducing the risk of infection from plasma products: Specific preventative strategies. Blood Rev. 2000;14: 94-110). Another suitable viral reduction method is viral removal by 15-35nm nanofiltration. This method improves the margin of safety in particular for non-enveloped viruses (Burnouf T, Radosevich M: Nanofiltration of plasma-derived biopharmaceutical products. Haemophilia. 2003;9: 24-37).

One major advantage of the process of the invention is that is can be carried out with small volumes of cryo-poor plasma with good yields. It can furthermore be carried out in a system of disposable single-use transfusion bags and disposable capsule filters. The process is therefore a valuable alternative to state of the art IgG preparation methods that require sophisticated blood fractionation facilities and thus opens new perspectives for countries without large-scale fractionation facilities to prepare IgG from domestic plasma at affordable costs. The process can also be useful for the treatment of so-called "convalescent plasma" collected from donors who have developed neutralizing IgG against infectious agents such as H1N1, H5N1, SARS, West Nile Virus, etc. Such convalescent plasma can be treated in the single use small scale processing system to isolate the immunoglobulins that can be used to treat patients infected by the same infectious agent.

In order to more fully illustrate the nature of the invention and the manner of practising the same, the following non-limiting examples are presented.

### EXAMPLE

### Preparation of IgG according to the invention

### Preparation of the plasma material and cryoprecipitation

Whole blood donations were collected by whole blood collection (Cairo, Egypt) from volunteer non-remunerated donors and sent to the Shabrawishi Hospital Blood Bank (Giza, Cairo, Egypt) for processing. Recovered plasma was prepared from whole blood, collected into CPD anticoagulant/preservative (ratio: 14ml/100 ml of blood). Blood was centrifuged at 3600 g for 12 min within 4hrs of collection. Plasma was not leucoreduced prior to processing. Plasma was frozen in a -30°C freezing room and stored in a freezer at -30°C or colder for a maximum of 12 months.

Plasma donations with anti-A and anti-B titers < 32 were then subjected to cryoprecipitation by thawing at 4°C overnight until the material becomes slushy. After centrifugation (model KR4i, Jouan, St Herblain, France) for 30 min at 3850 x g and -4°C, the cryo-poor plasma (about 200mL) was recovered into a transfer plastic transfusion bag and frozen at -30°C or colder.

### Recovery of IgG containing fraction

### a) from DEAE-Sephadex supernatant

20 cryo-poor plasma donations were thawed at 30-35°C. Each two units of thawed cryo-poor plasma were pooled aseptically into a 600 ml sterile transfer bag. Autoclaved DEAE-Sephadex A-50 (GE Healthcare) suspended in a little volume of sterile saline solution was aseptically added to each bag at a ratio of 3g/L of cryo-poor plasma and incubated in a shaker incubator at 150 rpm for 30 min at 31°C to adsorb the coagulation factors of the PCC (Factors II, VII, IX, X) and protein C and protein S. The chromatographic resin was removed aseptically by filtration on a 0.45 µm sterile filtration device and the DEAE-Sephadex A-50 supernatant about 400mL/bag)) was recovered. 10 pooling bags were prepared each containing about 400mL of DEAE-Sephadex supernatant.

### b) From cryo-poor plasma

20 cryo-poor plasma donations were thawed at 30-35°C. 2 units of cryo-poor plasma (400 mL) were pooled aseptically into a 600mL plastic bag. 10 pooling bags were prepared each containing about 400mL of cryo-poor plasma.

### Caprylic acid fractionation of IgG

Caprylic acid (Merck, Darmstadt, Germany) was added slowly to each bag of about 400 mL of the DEAE-Sephadex supernatants or cryo-poor plasma in their sterile plastic bags under constant stirring to a final concentration of 5% (v/v) and pH 5.5+/- 0.1. The mixture was incubated at 31+/- 0.5 C at 150 rpm in a temperature-controlled shaker-incubator (31 +/- 0.5°C) for 2 hours under continuous stirring. The precipitated proteins were removed by centrifugation using a blood bank centrifuge (Jouan) at 3500g for 45 min at 0-4°C. The clear supernatants (total of about 2.8 L) were aseptically pooled into a 4L PVC bag, concentrated to 28-30 g protein /L (corresponding to about 800 mL) using a sterile single-use hemodialyzer (F6HB, Fresenius, Bad Homburg, Germany), hemodialysis pump and monitoring equipment (Gambro). The solution was then diluted with 5 volumes of sterile pyrogen-free saline solution and subjected to further diafiltration/concentration using the same sterile single-use hemodialyzer (F6HB, Fresenius, Bad Homburg, Germany) to remove the caprylic acid and decrease the volume to about 800 mL. One additional centrifugation was performed to remove any particulate using a blood bank centrifuge (Jouan) at 3500g for 45 min at 2-4°C The concentrated IgG solution was then passed by gravity through a filtration sequence comprising a pyrogen-free pharmaceutical grade BC0025L60SP03A cartridge (3M Cuno, Cergy-Pontoise, France) and a 0.2 µm mini-kleenpack sterilizing filter (Pall Corporation, Dreieich, Germany) and was directly dispensed into sterile plastic storage bags.

### Characterization

### Protein assays

Samples were taken at the various steps of the process and frozen at - 80°C until protein assessment. Protein determinations were performed at the Shabrawishi Hospital Blood Bank, Cairo. Total protein was determined by Biuret (Dialab, Wien, Austria), IgG, IgM, and IgA by immunoassays (Nephstar, Goldsite, Shenzhen, China), and albumin by a photometric method using bromocresol green (DiaSys Diagnostic Systems, Holzheim, Germany).

### Determination of molecular size of the IgG:

The samples were analyzed by HPLC, equipped with the isocratic pump model SDS 9414 and UV-VIS detector model S3210, a Rhiodyne manual injector and the PeakSimple Chromatography Data System SRI Model333 as data integrator (Schemback, Germany). Size Exclusion Chromatography column TSKGel G3000SWXL (7.8mm ID X 30 cm L) with a TSKGel guard column (6.0mm ID X 4.0 cm L) supplied by SIGMA company was attached to the system. The samples were eluted using 0.1 M sodium sulfate, 50 mM sodium acetate, 0.05% sodium azide, pH5 at flow rate of 0.5 ml/min for 30 min and wave length 280nm.

### Caprylic acid

1 mL samples were extracted directly by partitioning using n-hexane. The extraction repeated five times and each hexane extract was collected in one container and dried using flow of nitrogen or air. Caprylic acid was determined by injecting 1 µl of the extracted solution online into a gas chromatograph (SRI 8610C, SRI, Torrance, California, USA) equipped with flame ionization detector, using nitrogen at 40 ml/min, 200°C, and a Chromosorb WHP column (2-m length, ID:2mm; liquid phase FFAP; mesh range 80/100). The recovery of the extraction procedure was over 90%, and the limit of detection was less than 50 µg/ml.

### Results

The IgG was obtained with a mean recovery of 50 to 65% from cryo-poor plasma, corresponding to about 4.5 to 6 g/L. The IgG was 95% pure or more. IgG was at a mean concentration close to 30 g/L and albumin was less than 3 g/L. IgA and IgM were 2.7 and 0.78 g/L, respectively. There was no detectable PKA nor proteolytic activity. High-molecular-weight proteins/aggregates were less than 3% and monomers + dimers more than 90 %. Residual caprylic acid in the final preparation was less than 50 ppm.

These results clearly show that the process of the invention allows preparing IgG in small amounts in an efficient and inexpensive way. This is of particular value in countries that cannot afford a sophisticated manufacturing pharmaceutical facility or under situations where plasma from convalescent donors should be used to treat patients infected by a pathogen. In addition, the process is flexible and allows for a progressive up-scale to treat increasing volume of plasma when it becomes available in larger quantities.The process of the invention thus opens new perspectives for countries without large-scale fractionation facilities to prepare IgG from domestic plasma at affordable costs.

## Claims

1. Process for obtaining immunoglobulin G comprising the steps of:
a/ treating cryo-poor plasma with caprylic acid ;
b/ centrifugation of the caprylic acid mixture of step a/ and recovery of the supernatant;
c/ removing caprylic acid by diafiltration/concentration from the supernatant of step b/;
d/ filtrating by gravity the product obtained in step c/ above.

2. Process for obtaining immunoglobulin G comprising the steps of
- incubating cryo-poor plasma with an anion exchanger;
- treating the supernatant recovered in the previous step with caprylic acid;
- centrifugation of the caprylic acid mixture and recovery of the supernatant
- removing caprylic acid by diafiltration/concentration from the supernatant of the previous step;
- filtrating by gravity the product obtained in the previous step.

3. Process according to claim 1 or 2, wherein the caprylic acid treatment step is carried out at a pH between 4.7 and 6, preferably at pH 5.5.

4. Process according to anyone of claims 1 to 3, wherein the diafiltration is carried out on a sterile hemodialyser with a molecular weight cut-off of 30kDa.

5. Process according to claim 4, wherein the hemodialyser is a single-use hemodialyser.

6. Process according to anyone of claims 1 to 5, wherein the cryo-poor plasma is human cryo-poor plasma..

7. Process according to anyone of claims 1 to 6 further comprising, after the step of filtrating by gravity, a step of virally inactivating the IgG obtained after the filtration by gravity.

8. Immunoglobulin G (IgG) obtained by the process according to anyone of claims 1 to 7.
